(19) **Europäisches Patentamt**

**European Patent Office**

**Office européen des brevets**

(11) Veröffentlichungsnummer: **0 274 708 B1**

(12) # EUROPÄISCHE PATENTSCHRIFT

(45) Veröffentlichungstag der Patentschrift: **13.05.92**

(21) Anmeldenummer: **87118811.6**

(22) Anmeldetag: **18.12.87**

(51) Int. Cl.⁵: **A61K 45/06**, A61K 31/70, A61K 35/78, //(A61K35/78, 31:70,31:165)

(54) **Mittel zur Verabreichung bei Hypoglykämie.**

(30) Priorität: **23.12.86 DE 3644092**

(43) Veröffentlichungstag der Anmeldung:
**20.07.88 Patentblatt 88/29**

(45) Bekanntmachung des Hinweises auf die Patenterteilung:
**13.05.92 Patentblatt 92/20**

(84) Benannte Vertragsstaaten:
**AT BE CH DE ES FR GB IT LI NL SE**

(56) Entgegenhaltungen:

**CHEMICAL ABSTRACTS, Band 82, Nr. 19, 12. Mai 1975, Seite 53, Zusammenfassung 119151h, Columbus, Ohio, US; J.M. RODRIGO GOMEZ et al.: "Effect of metoclopramide on gastric emptying of water and a 10% glucose solution" & REV. ESP. ENFERM. APAR. DIG. 1974, 44(5), 469-84**

(73) Patentinhaber: **Bergis, Kristian, Dr.**
**Theodor-Klotzbücher-Strasse 10**
**W-6990 Bad Mergentheim(DE)**

Patentinhaber: **Boerner, Harro**
**Kleinfeldstrasse 41 A**
**W-8100 Garmisch-Partenkirchen(DE)**

Patentinhaber: **Ouadbeck, Jürgen**
**von Berlichingen-Strasse 17**
**W-6990 Bad Mergentheim(DE)**

(72) Erfinder: **Bergis, Kristian, Dr.**
**Theodor-Klotzbücher-Strasse 10**
**W-6990 Bad Mergentheim(DE)**
Erfinder: **Boerner, Harro**
**Kleinfeldstrasse 41 A**
**W-8100 Garmisch-Partenkirchen(DE)**
Erfinder: **Ouadbeck, Jürgen**
**von Berlichingen-Strasse 17**
**W-6990 Bad Mergentheim(DE)**

CHEMICAL ABSTRACTS; Band 84, Nr. 25, 21. Juni 1976, Seite 16, Zusammenfassung 173587j, Columbus, Ohio, US; S. KITAZAWA et al.: "Effect of glucose on drug absorption from the small intestine" & PROC. SYMP. DRUG METAB. ACTION, 4th 1972, (Publ. 1973), 11-24

ROTE LISTE, 1985, Zusammenf. 59193, Editio Cantor, Aulendorf/Württ., DE, "Digestodoron"

ROTE LISTE, 1985, Zusammenf. 59194, Editio Cantor, Aulendorf/Württ., DE, "Iberogast"

(74) Vertreter: **Patentanwälte Phys. Bartels Dipl.-Ing. Fink Dr.-Ing. Held Lange Strasse 51 W-7000 Stuttgart 1(DE)**

## Beschreibung

Die Erfindung betrifft die Verwendung von Glucose und einem chemischen und/oder pflanzlichen Motilitätsförderer zur Herstellung eines Arzneimittels.

Zusammensetzungen, die Glucose und einen Motilitätsförderer enthalten, sind bekannt (Chemical Abstracts, Band 84, 1976, Zusammenfassung Nr. 173587j). Es ist ferner bekannt, die Wirkung von Metoclopramid auf die Entleerung von Wasser aus dem Magen einer gesunden Person mit Hilfe einer 10%igen Glucoselösung auszuschließen (Chemical Abstracts, Band 82, 1975, Zusammenfassung Nr. 119151h) und mit Hilfe von Glucose die Absorption von Metoclopramid im Dünndarm einer Person zu erhöhen (Chemical Abstracts, Band 84, 1976, Zusammenfassung Nr. 173587j).

Weiterhin ist es bekannt, Diabetikern Glucose im Falle einer Hypoglykämie zu verabreichen. Diabetiker erleiden nämlich selbst bei guter Stoffwechselführung mehr oder minder häufig eine Hypoglykämie oder Unterzuckerung, die eine Kohlenhydratzufuhr erforderlich macht. Obwohl die Diabetiker in der Regel die Symptome einer Unterzuckerung kennen und wissen, daß eine sofortige Kohlenhydratzufuhr notwendig ist, kommt es immer wieder bei Diabetikern zu starken Überzuckerungen, mit denen eine Selbstgefährdung des Diabetikers, aber auch eine Fremdgefährdung verbunden sein kann.Dies ist zum einen auf die starke Dynamik der Hypoglykämie zurückzuführen, die den Blutzucker innerhalb kürzester Zeit dramatisch tief absinken lassen kann. Für einen Diabetiker sollte deshalb ein Kohlenhydrat, vorzugsweise Glucose, immer kurzfristig zur Verfügung stehen. Eine Gefahr besteht aber auch darin, daß die Wirkung des verabreichten Kohlenhydrats nicht rasch genug eintritt und der Diabetiker deshalb eine zu große Kohlenhydratmenge zu sich nimmt. Dies kann zu einer gefährlichen Überkorrektur, auch aufgrund einer ausgeprägten Gegenregulation, führen, was ebenfalls vermieden werden sollte.

Der Erfindung liegt daher die Aufgabe zugrunde, ein Arzneimittel zur Verabreichung bei Hypoglykämie von an Diabetes leidenden Personen zu schaffen, das die negativen Auswirkungen einer Hypoglykämie zumindest weitgehend zu beseitigen vermag. Diese Aufgabe löst ein Mittel gemäß dem Patentanspruch 1.

Die Kombination von Glucose mit einem Motilitätsförderer hat bei der Behandlung von Hypoglykämie zur Folge, daß auch bei Patienten mit autonomer diabetischer Neuropathie und Gastroparese sehr rasch, nämlich bereits nach etwa drei bis fünf Minuten, die Glucoseresorption erfolgt und unmittelbar darauf der Blutzuckeranstieg einsetzt. Außerdem wird ebenfalls kurzfristig die Motilität gesteigert, wodurch ein Funktionsmangel bestimmter Hirnstrukturen, der bei niedrigen Blutzuckerwerten auftreten und zu einem Bewußtseinsverlust mit Beeinträchtigung der Motorik führen kann, vermieden oder rasch wieder beseitigt wird. Dies gilt auch für die psychischen Fehlreaktionen und die Gefahren für das zentrale Nervensystem, die bei einer Unterzuckerung auftreten können.

Als chemische Motilitätsförderer kommen beispielsweise Metoclopramid, Bromoprid, Domperidon oder Alizaprid in Frage, deren Nebenwirkungen jedoch berücksichtigt werden müssen.

Als phytotherapeutische oder pflanzliche Motilitätsförderer sind Medikamente auf der Basis von Iberis amara , also beispielsweise Iberogast, oder Digestodoron geeignet.

Um einen ausreichenden Blutzuckeranstieg zu gewährleisten, jedoch eine Überkorrektur zu vermeiden, ist es zweckmäßig, die Glucosemenge auf etwa 20 g zu begrenzen. Der Motilitätsförderer wird vorzugsweise in einer Einzeldosismenge beigegeben, die bei den chemischen Motilitätsförderern im Bereich bis zu 10 mg liegt.

Im Hinblick auf eine einfache Aufbewahrung und Einnahmemöglichkeit ist eine feste, pulverförmige oder flüssige Form des erfindungsgemäßen Arzneimittels vorteilhaft. Die flüssige Form hat den besonderen Vorteil der raschesten und besten Resorption. Aber auch Tabletten und Dragees, die sich ebenfalls gut aufbewahren lassen, so daß sie der Diabetiker problemlos stets bei sich führen kann und die auch einfach zu verabreichen sind, können eine in vielen Fällen noch ausreichend rasche Resorption haben.

Als Verpackung kommen vor allem Schachteln, Beutel und Ampullen in Frage. Dabei wird es in der Regel nicht erforderlich sein, die beiden Komponenten getrennt zu verpacken. Dies wäre nur dann erforderlich, wenn die beiden Komponenten bei einer Vereinigung chemisch reagieren würden. Besonders vorteilhaft dürfte eine Trinkampulle sein, die leicht zu öffnen ist und aus Kunststoff hergestellt sein kann, so daß keine Bruchgefahr besteht. Vorzugsweise ist bei einer solchen Trinkampulle die Halsweite so groß gewählt, daß der Inhalt ohne saugen zu müssen ausläuft und getrunken werden kann.

Das als eine Verpackungseinheit konfektionierte erfindungsgemäße Arzneimittels hat nicht nur den Vorteil, daß der Diabetiker die Verpackungseinheit problemlos stets bei sich führen kann. Vor allem gibt es dem Diabetiker die Sicherheit, das er selbst dann, wenn eine gewisse Bewußtseinsbeeinträchtigung schon eingetreten sein sollte, er die richtige Menge zu sich nimmt und daß die für die Einnahme erforderlichen Vorbereitungen keine Fingerfertigkeit notwendig machen und deshalb noch problemlos ausgeführt werden können.

Im folgenden ist die Erfindung anhand eines Beispiels erläutert.

In einer aus Kunststoff unter hohen Temperaturen und aseptischen Bedingungen hergestellten Trinkampulle sind 20 g Glucose und 5 bis 10 mg Metoclopramid in 15 bis 20 ml Wasser gelöst. Die Trinkampulle hat damit eine Größe, die es ermöglicht, daß eine Diabetiker sie ständig bei sich führt. Durch den hygienisch dichten Verschluß der Trinkampulle läßt sich die in ihr enthaltene Lösung langfristig dauerhaft stabilisieren. Treten Symptome einer Hypoglykämie wie zum Beispiel Schwitzen, Herzklopfen und Zittrigkeit, Schwindel, Sehstörung, Kopfdruck, Hunger oder Pelzigkeit auf, öffnet der Diabetiker die Trinkampulle und trinkt den Inhalt. Nach etwa drei bis fünf Minuten erfolgt die Resorption. Hierdurch bedingt ergibt sich ein Blutzuckeranstieg von ca. 50 bis 80 mg/dl. Damit wird eine Überreaktion verhindert.

Sehr gute Ergebnisse wurden bei Versuchen auch mit einem Zusatz von 1 ml der handelsüblichen Paspertin-Tropflösung als Motilitätsförderer zu 20 g Glukose in 60 ml $H_2O$ erzielt.

**Patentansprüche**

1. Verwendung von Glucose und einem chemischen und/oder pflanzlichen Motilitätsförderer zur Herstellung eines Arzneimittels zur Behandlung von Hypoglykämie von an Diabetes leidenden Personen.

2. Verwendung nach Anspruch 1, dadurch gekennzeichnet, daß es sich bei dem chemischen Motilitätsförderer um Metoclopramid, Bromoprid, Domperidon oder Alizaprid handelt.

3. Verwendung nach Anspruch 1, dadurch gekennzeichnet, daß es sich bei dem pflanzlichen Motilitätsförderer um ein auf der Basis von Iberis amara hergelltes Medikament oder um Digestodoron handelt.

4. Verwendung nach einem der Ansprüche 1 bis 3, dadurch gekennzeichnet, daß der chemische und/oder pflanzliche Motilitätsförderer in einer für eine Einzeldosis notwendigen Menge beigegeben ist.

5. Verwendung nach einem der Ansprüche 1 bis 4, dadurch gekennzeichnet, daß das Arzneimittel bei einer Einzeldosis etwa 20 g Glucose enthält.

6. Verwendung nach einem der Ansprüche 1 bis 5, dadurch gekennzeichnet, daß sich das Arzneimittel im flüssigen, pulverförmigen oder festen Zustand befindet.

7. Verwendung nach Anspruch 6, dadurch gekennzeichnet, daß das Arzneimittel in Form von Tabletten oder Dragees konfektioniert ist.

8. Verwendung nach einem der Ansprüche 1 bis 7, dadurch gekennzeichnet, daß das Arzneimittel in einer Verpackungseinheit konfektioniert ist.

9. Verwendung nach Anspruch 8, dadurch gekennzeichnet, daß es sich bei der Verpackungseinheit um eine Schachtel, einen Beutel oder eine Ampulle, insbesondere eine Trinkampulle, handelt.

10. Verwendung nach Anspruch 9, dadurch gekennzeichnet, daß die Ampulle aus Kunststoff besteht.

**Claims**

1. Use of glucose and a chemical and/or vegetable motility promoter for the preparation of a medicament for the treatment of hypoglycaemia in persons suffering from diabetes.

2. Use according to Claim 1, characterised in that the chemical motility promoter is metoclopramid, bromoprid, domperidon or alizaprid.

3. Use according to Claim 1, characterised in that the vegetable motility promoter is a medicament prepared from Iberis amara or is digestodoron.

4. Use according to any one of Claims 1 to 3, characterised in that the chemical and/or vegetable motility promoter is added in an amount necessary for a single dose.

5. Use according to any one of Claims 1 to 4, characterised in that a single dose of the medicament contains approximately 20 g of glucose.

6. Use according to any one of Claims 1 to 5, characterised in that the medicament is in the liquid, powder or solid state.

7. Use according to Claim 6, characterised in that the medicament is produced in the form of tablets or dragees.

8. Use according to any one of Claims 1 to 7, characterised in that the medicament is presented as a packaged unit.

**9.** Use according to Claim 8, characterised in that the packaged unit is a packet, a sachet or an ampoule in particular an ampoule for oral administration.

**10.** Use according to Claim 9, characterised in that the ampoule is made from plastics material.

**Revendications**

**1.** Application de glucose et d'un agent chimique et/ou végétal favorisant la motilité, à la préparation d'un médicament pour le traitement de l'hypoglycémie chez les personnes souffrant de diabète.

**2.** Application selon la revendication 1, caractérisée en ce que l'agent chimique favorisant la motilité est le Metoclopramide, le Bromopride, le Dompéridon, ou l'Alizapride.

**3.** Application selon la revendication 1, caractérisée en ce que l'agent végétal favorisant la motilité est un médicament préparé à base d'Iberis amara ou le Digestodoron.

**4.** Application selon l'une des revendications 1 à 3, caractérisée en ce que l'agent chimique et/ou végétal favorisant la motilité est administré en la quantité nécessaire pour une dose unitaire.

**5.** Application selon l'une des revendications 1 à 4, caractérisée en ce que le médicament contient pour une dose unitaire environ 20 g de glucose.

**6.** Application selon l'une des revendications 1 à 5, caractérisée en ce que le médicament se trouve à l'état liquide, pulvérulent ou solide;

**7.** Application selon la revendication 6, caractérisée en ce que le médicament est confectionné sous forme de comprimés ou de dragées.

**8.** Application selon l'une des revendications 1 à 7, caractérisée en ce que le médicament est confectionné en un conditionnement unitaire.

**9.** Application selon la revendication 8, caractérisée en ce que le conditionnement unitaire est une boîte, un sachet ou une ampoule, en particulier une ampoule buvable.

**10.** Application selon la revendication 9, caractérisée en ce que l'ampoule est faite de matière synthétique.